(19) Europäisches Patentamt
European Patent Office
Office européen des brevets

(11) EP 0 699 468 B1

(12) # EUROPEAN PATENT SPECIFICATION

(45) Date of publication and mention of the grant of the patent: **11.03.1998 Bulletin 1998/11**

(51) Int. Cl.[6]: **B01D 53/14**, C07C 7/156

(21) Application number: **95305762.7**

(22) Date of filing: **17.08.1995**

(54) **Removal of olefins from fluids**

Verfahren zur Entfernung von Olefinen aus Fluiden

Procédé pour l'élimination d'oléfines de fluides

(84) Designated Contracting States:
**BE DE FR GB NL**

(30) Priority: **29.08.1994 US 297339**

(43) Date of publication of application:
**06.03.1996 Bulletin 1996/10**

(73) Proprietor: **BP Chemicals Limited**
**London EC2M 7BA (GB)**

(72) Inventors:
- **Davis, James Critsen**
**Hudson, Ohio 44236 (US)**
- **Valus, Ronald James**
**Valley View, Ohio 44125 (US)**

(74) Representative:
**Krishnan, Suryanarayana Kalyana et al**
**BP INTERNATIONAL LIMITED**
**Patents & Agreements Division**
**Chertsey Road**
**Sunbury-on-Thames Middlesex TW16 7LN (GB)**

(56) References cited:
**US-A- 3 514 488**
**US-A- 3 801 666**
**US-A- 5 259 986**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 699 468 B1

## Description

## Background of the Invention

This invention relates to the removal of olefinic gases, particularly ethylene, from mixtures thereof with other saturated and unsaturated hydrocarbon gases by bringing the mixture of gases into contact with an absorbent capable of selectively absorbing the olefinic gases.

It is well known that removal of olefinic gases from a mixture thereof with other hydrocarbons can be facilitated by absorption in suitable solutions capable of complexing such olefins. Particularly well known are complexing agents comprising a solution of silver nitrate, or, a solution of cuprous salts combined with a ligand.

The use of silver nitrate solutions has the problem that although it is very good at separating olefins from non-olefinic hydrocarbon gases, it is virtually impossible to separate the olefins from one another. Moreover, the use of silver nitrate solutions is significantly less economic due to cost of such solutions.

Processes involving the use of cuprous salts are described extensively, for instance, in US-A-2245719, US-A5104570 and the Proceedings of the Sixth World Petroleum Congress, Frankfurt am Main, June 19-26, 1963, Section IV, Paper 14-PD8, pages 325-343, entitled "Base stocks from Petroleum and Natural Gas for the Chemical Industry" and published by Verein zur Förderung des 6.Welt-Erdöl-Kongresses, Hamburg. Taking these references individually:

US-A-2245719 describes the separation and concentration of olefins from gaseous mixtures containing olefins and particularly to separation and concentration of ethylene and propylene from mixtures thereof with saturated and unsaturated hydrocarbons. The gaseous mixture comprising the olefins is brought into contact with cool solutions of cuprous salts and liquid organic nitrogen compounds. The solutions specifically disclosed are (a) one prepared with 4 moles of pyridine, 2 moles of acetic acid per liter and saturated with cuprous oxide to form a complex so that at 25°C, the solution contains about 1.92 moles of cuprous copper, and (b) another which is an aqueous solution containing 3 moles of pyridine, 6 moles of hydrochloric acid per litre and saturated with cuprous chloride. No other anions forming a cuprous salt is disclosed. It is stated that these complexes could be used as solutions in combination with e.g. isopropyl alcohol.

One of the problems with this type of formulation is that such solutions which contain acetic acid do not have sufficient uptake of olefins whereas solutions containing hydrochloric acid are highly corrosive. Thus, there is room for significant improvement over the complexes described in this reference.

The Sixth World Petroleum Congress article is primarily directed to the use of ammoniacal copper solutions and ethanolamine-Cu(I)-nitrate solution containing 175 g copper(I)/litre at 20°C for ethylene up to 1200 kpa. Discussing the influence of anion and complex-formers on absorption, this article states that a majority of patent specifications are based on ammoniacal copper(I) salt solutions which eliminate the corrosion problem of hydrochloric acid solutions. It is further stated that in most cases, the use of carbonate, formate or acetate is suggested and other specifications mention nitrate, salicylate, phenolate, cresolate and benzene sulphonate as alternative anions in such ammoniacal solutions. This article goes on to state on page 327 that:

"The main substitutes for ammonia [in the ammoniacal copper(I) solutions] are hydroxyalkylamines, e.g. ethanolamines, propanolamines and butanolamines. Among organic nitrogen compounds, pyridine, piperidine, formamide, acetamide[15] and methylamine[16] have also been proposed."

Reference 15 identified in this passage corresponds to US-A-2245719 reviewed above. This article describes at page 336 an olefins separation plant for the separation of cracked gases with ethanolamine copper nitrate solution and particularly refers to Gendorf works of Hoechst A G in Germany. Reverting to page 333 of this article, it is made clear in Section 3.14 under "Corrosion problems and materials" that "Brass and other materials containing copper should be avoided as it is well known that copper is dissolved by ammonia or ammonia derivatives."

One of the disadvantages of this process is that copper(I) complexes of ethanolamine absorb gases such as carbon monoxide and hence are less efficient at absorbing olefins for the simple reason that the active sites for absorption of olefins are used up by carbon oxides. In any event, this reference makes no mention of the use of a cuprous nitrate • water • pyridine type system for removal of olefins from mixtures thereof with other hydrocarbon gases.

US-A-5104570 relates to olefin complexing agents comprising Cu(I) carboxylate/$BF_3$ adducts in aromatic solvents for separating olefins from paraffins. Although this application was filed on December 26, 1989, a long time after the above two documents were published, it nevertheless points out in column 1, lines 27-33 that:

"aqueous systems involving Cu(I) salts and ammonia or ammonium are corrosive and lack necessary long term stability. Non-aqueous Cu(I) solutions using a pyridine solvent have proven difficult to handle due to the solvent and require large scale systems because the reagent is in the form of a slurry in the solvent."

This implies that non-aqueous systems are preferred to minimise corrosion and long-term storage stability problems but that such systems using pyridine as the ligand are difficult to handle.

A series of papers appeared in the 1970's by a group in Australia using nitrile-stabilized copper(I) as a means of electrorefining copper (Muir, D. M., Parker, A. J., Waghorne, W. E., Hydrometallurgy, Vol. 1, pp 61-77, 1975; Muir, D. M.,

Parker, A. J., Sharp, J. H., Waghorne, W. E., Hydrometallurgy, Vol. 1, pp155-168, 1975; MacLeod, I. D., Muir, D. M., Parker, A. J., Singh, P., Australian Journal of Chemistry, Vol. 30, pp 1423-1437, 1977; Parker, A. J., Clarke, D. A., Couche, R. A., Miller, G., Tilley, R. I., Waghorne, W. E., Australian Journal of Chemistry, Vol. 30, pp 1661-72, 1977). These reference discuss the instability of the cuprous ion in aqueous solutions, describing how copper (I) ions readily disproportionate via the following reaction:

$$2Cu^{+} \leftrightarrow Cu^{0} + Cu^{+2} \qquad \text{(Equation 1)}$$

The papers also note that copper (I) ion is better solvated by certain organic ligands, such as nitriles, than by water. With the addition of at least four moles of acetonitrile per mole of $Cu^{+}$ to water, the equilibrium constant for Equation 1 changes from approximately $10^{6}$ in water to about $10^{-10}$ in acetonitrile/water mixtures. Stable, colorless cuprous salts were found to be stable even at 3 molar concentrations, provided that at least 2-4 moles of acetonitrile was present per mole of copper. The volatile acetonitrile can be replaced with 3-hydroxypropionitrile (b.p. 228°C) with little loss of performance.

Similarly, US-A-3801666 claims and describes an anhydrous solution of cuprous nitrate in propionitrile solvent containing about 15 to 26% by weight of cuprous ion and no more than 2 to 5% by weight of cupric ion, a process for producing the same and the use of such anhydrous solutions for separating mixtures of closely boiling aliphatic hydrocarbons having from 2-10 carbon atoms consisting essentially of at least two hydrocarbons of different degrees of unsaturation by selectively complexing a more highly unsaturated hydrocarbon with a cuprous ion containing copper salt in non-aqueous solvent and subsequently decomposing the complex to recover the hydrocarbon therefrom.

**Summary of the Invention**

It is, therefore, an object of the present invention to achieve removal of olefins from mixtures containing olefins by bringing the mixture into contact with an absorbent capable of selectively absorbing the olefin.

Another object of the present invention is a process for olefin separation that avoids the use of highly corrosive acids or caustics. A related object of the present invention is the utilisation of copper(I) compounds for olefin separation. Yet another object of the present invention is the stabilisation and improved handling of copper(I) compounds. Additionally, an object of the present invention is the *in situ* formation of copper(I) complexes by passing a solution containing copper(II) complexes over metallic copper, i.e. copper(0).

To achieve the foregoing objects and in accordance with the purpose of the invention, as embodied and broadly described herein, the process of the invention comprises removal of an olefin from a fluid, comprising

(a) contacting a first solution containing copper (II) species with metallic copper to form an aqueous second solution containing copper (I) complexes wherein said second solution contains a complexing ligand selected from the group consisting of pyridine, methyl substituted pyridines, methoxy substituted pyridines, and compounds represented by the formulae

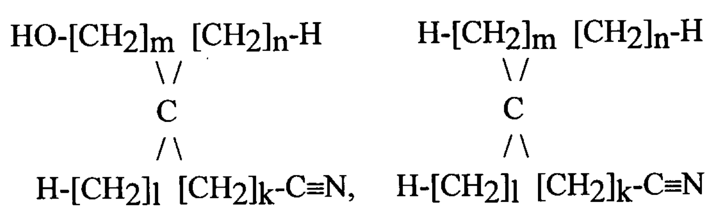

where k = 0 to 4, l = 0 to 4, m = 0 to 4, n = 0 to 4, and combinations of the same; and

(b) contacting the aqueous second solution containing copper (I) complexes with the olefin-containing fluid to remove a portion of the olefin from the fluid.

The value of k + l + m + n = 0 to 4, and preferably = 0 to 2 .

Thus, the complexing aqueous second solution suitably contains a complexing ligand selected from the group consisting of pyridine, acetonitrile, 3-hydroxypropionitrile, and combinations of the same.

It is preferred that the copper(I) species in the aqueous second solution is selected from the group consisting of cuprous acetate, cuprous nitrate, cuprous sulphate, and combinations of the same.

**Description of Drawings and Figures**

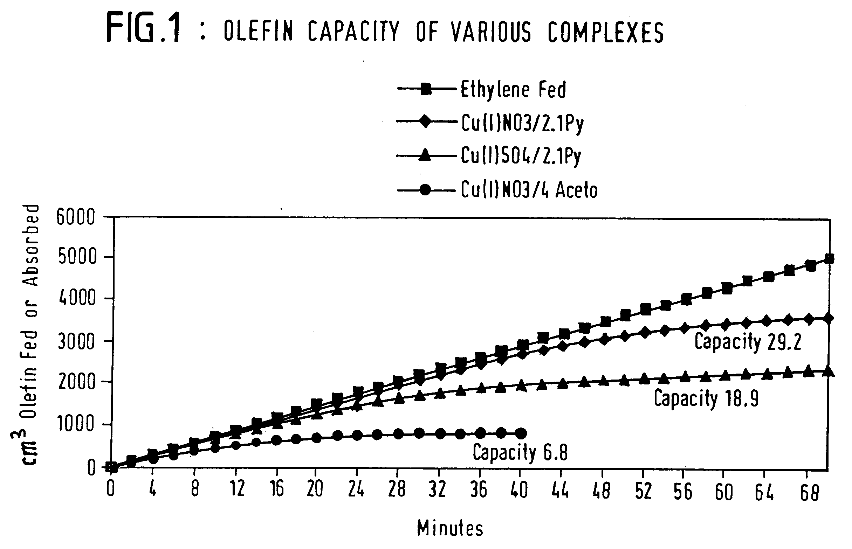

Figure 1 is a chart depicting the olefin capacities of various copper(I) complexes.

**Detailed Description of the Invention**

It has now been found that the above problems can be mitigated by using a unique combination of copper(I) anion and ligand in aqueous solution.

Accordingly, the present invention is a process for the removal of C2/C3 olefins from a mixture of gases comprising saturated and unsaturated hydrocarbons said process comprising bringing said mixture of gases into contact with a aqueous solution of a complex salt of cuprous nitrate and a complexing ligand said ligand being selected from the group consisting of diethylene triamine, pyridine, acetonitrile, hydroxypropionitrile and derivatives thereof, wherein the molar ratio of the ligand to cuprous ion is in the range from 1:1 to 6:1 and the aqueous solution comprises 1-4 gram moles of the complex salt per litre of water.

The complex salts used to remove C2/C3 olefins may either be preformed, or made immediately prior to the absorption step. The generation of the complex salt in situ gets round the problem of the lack of stability of such complexes in the presence of air. Thus, a $Cu(I)NO_3 \cdot$ pyridine complex salt can be made by mixing in a reaction vessel $Cu(NO_3)_2$ with a aqueous solution of pyridine and then adding copper metal, which has previously been washed with, for example, a trisodium salt of ethylene diamine tetraacetic acid to remove any oxide layers on the copper metal. The mixture contained in the reaction vessel is kept under a blanket of nitrogen. Alternatively, a Cu(II)nitrate • pyridine complex can be passed over a bed of copper shot under nitrogen to convert the Cu(II) salt to the Cu(I) form *in situ* immediately prior to the absorption step. The generation of the complex salt in situ gets round the problem of the lack of stability of such complexes in the presence of air.

The mole ratio of the complexing ligand to the cuprous ion in the complex salt is suitably in the range from about 1:1 to 6:1, preferably about 2-4:1, most preferably about 2-3:1. The aqueous solution comprises 1-4 gram moles of the complex salt per litre of water, preferably 1.75 to 3.25 gram moles of the complex salt per litre of water, most preferably 2.0 to 3.0 gram moles of the complex salt per litre of water. Thus, the aqueous second solution has a concentration of copper(I) complexes between about 1M and about 6M, preferably between about 2M and about 3M.

The complex salt thus formed is brought into contact with mixture of gases comprising the desired C2/C3 olefins. However, any acetylenic compounds present in the mixture of gases to be treated are capable of forming acetylides which, particularly in the dry state, may decompose explosively. Whilst the copper acetylides are not as hazardous as the corresponding silver acetylides, nevertheless, acetylene binds very tightly with cuprous salts and thereby significantly reduce the efficiency of such complex salts to absorb C2/C3 olefins. It is desirable to render the mixture of gases substantially free of all acetylenic hydrocarbons before such gaseous mixture is brought into contact with the complex salt solution. Such removal of acetylenic compounds can be carried out, for example, by selective hydrogenation thereof to the corresponding olefins and similar processes known in the art. Alternatively, acetylenic and diene compounds present in the mixture of gases may be removed in a pre-treatment stage by passing said gases over a zeolite bed containing silver ions prior to contact with the solution of the complex salt. The amount of acetylenic hydrocarbons in the mixture of gases should suitably be reduced to below 20 ppm, preferably below 10 ppm, and most preferably below 1 ppm, prior to contact with the complex salt solution.

The mixture of gases - substantially free of acetylenic hydrocarbons - is brought into contact with a solution of the complex salt. Suitable contacting temperatures are in the rage from about 0°C to 100°C, preferably from about 15°C to about 35°C, most preferably from about 20°C to about 30°C. Suitable contacting pressures are in the rage of about 80 kpa to 3000 kpa, preferably from about 600 kpa to about 1800 kpa, most preferably from about 950 kpa to 1400 kpa.

The absorbed C2/C3 olefins can be recovered by venting the complex salt solution to reduce the pressure and/or by slightly increasing the temperature thereof to above the absorption temperature thereby releasing the absorbed olefins.

The complex salt solutions of the present invention have the following advantages:

a. The complex salts are stable in aqueous solutions, especially when using weakly basic ligands such as acetonitrile, pyridine and hydroxypropionitrile.

b. The complex salt solutions of the present invention have a high uptake of olefin per Cu(I) which is contrary to previous belief of poor uptake on Cu(I) and which belief in turn may have been due to unstable complex salts and/or poor choice of anion and complexing ligands.

c. The complex salts now used have a optimum olefin absorption capacity when the ligand to copper mole ratio of around 2-3:1.

**Experimental Results**

**Example 1**

Approximately 125 $cm^3$ of 2.0M $CuNO_3 \cdot 2.1$ pyridine was prepared from copper(II) and copper(0) precursors, forming a copper(I) complex via equation (1) above. Copper powder (10 μm particle size) was cleaned free of the oxide coating by stirring in a aqueous solution of trisodium EDTA, rinsed with distilled water and acetone, and dried. 7.9 g of the resulting copper powder was charged to a nitrogen-blanketed 250 ml reaction flask. A solution of 32.5 g $Cu(NO_3)_2 \cdot 2.5$ $H_2O$ (0.137 moles, 10% excess) dissolved in 82.5 ml distilled water was added. The mixture was stirred under nitrogen for 5 minutes. 41.6 g of pyridine was added and the solution was stirred overnight. The maximum total copper calculated was 0.25 moles, the calculated copper concentration was 2.0 M, and the amine/Cu ratio was calculated to be 2.1.

The olefin absorption capacity of the solution was measured by bubbling ethylene in below the surface of the copper complex solutions. The 2.5 M $CuNO_3 \cdot 2.1$ pyridine solution was charged to a 250 ml three-neck flask positioned over a magnetic stirring. A feed gas of pure ethylene was metered using a mass flow controller. The outlet of the mass flow controller was connected to a small diameter tube which was positioned in one neck of the flask so that the olefin-containing feed gas was forced to bubble through the solution. The effluent gas exited the flask through a second neck of the flask and was directed to a wet test meter. The neck openings around the gas inlet and gas outlet were sealed, as was the third, unused, opening. An egg-shaped magnetic stirring bar, driven at high speed by the magnetic stirring plate, assured intimate contact between the olefin-containing feed gas and the solutions tested.

The effluent gas from the flask was manually monitored with a wet test meter every two minutes. Testing of the solution was terminated at either 70 minutes or when the rate of effluent flow equalled the rate of feed for several readings.

The olefin capacity of solution was calculated as the volume of olefin absorbed per unit volume of solution (i.e., $cm^3$ gas absorbed per $cm^3$ of solution). The difference between the feed gas and effluent gas volumes was assumed to be the gas absorbed.

The olefin capacity of the solution is reported as Experiment Number 1 in Table 1, below. The results are reported as Experiment Number 1 in Table 1.

**Examples 2-17**

Solutions of various anions, ligands and ligand/metal ratios were prepared and evaluated by the methods described above. Solutions were prepared according to the method of Example 1. The olefin capacities of the solutions are reported as Experiments 2-17 in Table 1.

**Example 18**

A solution of 2.0 M $CuNO_3 \cdot 2.5$ pyridine was prepared from copper(II) and copper(0) precursors, similar to Example 1, except the copper(0) metal was maintained in a separate vessel to simulate *in situ* generation of the copper(I) complex. 38.7g $Cu(NO_3)_2 \cdot 2.5$ $H_2O$ (0.137 moles, 10% excess) dissolved in 82.5 ml distilled water was placed in a nitrogen-blanketed 250 ml reaction flask. The mixture was stirred under nitrogen for 5 minutes. 49.5 g of pyridine was added, and stirring was maintained. 2 mm diameter copper shot was cleaned free of the oxide coating by stirring in an aqueous solution of trisodium EDTA, rinsed with distilled water and acetone, and dried. 9.4 g of the resulting copper shot was charged to a second nitrogen-blanketed 250 ml reaction flask. Transfer pumps were used to circulate the $Cu(NO_3)_2$/pyridine solution from the first reaction flask to the second reaction flask, and back again, in a closed-loop. Circulation was maintained for three days, and it was observed that the solution had turned from deep blue to yellow. The olefin capacity of the solution was tested as described above, and is reported as Experiment 18 in Table 1.

**Example 19**

Comparative stability of copper(I) solutions to hydrogen were evaluated. A 2.0 M $CuNO_3 \cdot 2.2$ pyridine was prepared according to the procedure of Example 1, and hydrogen gas was bubbled through the resulting solution at a rate of 20 $cm^3$/min for 75 hours. No visible reduction of the copper metal was observed.

**Example 20**

An 2.0 M aqueous $AgNO_3$ solution was prepared, and hydrogen gas bubbled through the resulting solution in the manner described in Example 19. Precipitation of black solid, finely dispersed silver metal was observed within 15 minutes.

**Examples 21-23**

250 ml samples of solutions prepared in the manner of Examples 1, 10 and 16 above. The sample solution was placed in a flask, and ethylene gas was bubbled through the solution at a rate of approximately 70 standard $cm^3$ The effluent gas exited the flask through a second neck of the flask and was directed to a wet test meter. The neck openings around the gas inlet and gas outlet were sealed, as was the third, unused, opening. An egg-shaped magnetic stirring bar, driven at high speed by the magnetic stirring plate, assured intimate contact between the olefin-containing feed gas and the solutions tested.

The effluent gas from the flask was manually monitored with a wet test meter every two minutes, and the volumetric flow rate of the effluent gas recorded.

The olefin capacity of solutions was calculated as the volume of olefin absorbed per unit volume of solution (i.e., $cm^3$ gas absorbed per $cm^3$ of solution). The difference between the feed gas and effluent gas volumes was assumed to be the gas absorbed.

The olefin capacities of the solutions are reported in Figure 1, below. Also included in Figure 1 is the cumulative amount of olefin fed to the flask. The proximity of the absorption curve to the feed curve is a qualitative measure of the kinetics of the olefin uptake. The solutions with high capacities absorb virtually all the olefin up to approximately half their total capacity before the absorption rate begins to fall off.

TABLE 1

**CUPROUS COMPLEXES PREPARED AND THEIR OLEFIN CAPACITIES**

| Expt. No. | Cuprous salt | Molar conc. | Ligand | Ligand/Cu | Capacity $cm^3$ olefins/$cm^3$ |
|---|---|---|---|---|---|
| | | | | Mol Ratio | facilitator solution |
| 1 | nitrate | 2.0 | pyridine | 2.1 | 29.2 |
| 2 | sulphate | 2.0 | acetonitrile | 4 | 6 |
| 3 | acetate | 2.0 | pyridine | 2.0 | 14.6 |
| 4 | sulphate | 3.0 | acetonitrile | 4.0 | 7.5 |
| 5 | sulphate | 3.0 | acetonitrile(1) | 4.0 | 8.6 |
| 6 | acetate | 2.0 | pyridine | 2.0 | 15 |
| 7 | acetate | 3.0 | pyridine | 2.0 | 13.3 |
| 8 | nitrate | 2.0 | pyridine | 2.5 | 23.7 |
| 9 | nitrate | 2.0 | acetonitrile | 4.0 | 6.9 |
| 10 | nitrate | 2.0 | acetonitrile | 4.0 | 6.8 |
| 11 | acetate | 2.0 | acetonitrile | 2.5 | 3.4 |
| 12 | nitrate | 2.0 | acetonitrile | 4.0 | 6.6 |
| 13 | nitrate | 2.0 | acetonitrile | 6.0 | 2.2 |
| 14 | nitrate | 2.0 | acetonitrile | 2.1 | 20.9 |
| 15 | nitrate | 2.0 | acetonitrile | 3.0 | 12.8 |
| 16 | sulphate | 2.0 | pyridine | 2.1 | 18.9 |
| 17 | sulphate | 2.0 | hydracrylonitrile(2) | 2.1 | 16.7 |
| 18 | nitrate | 2.0 | pyridine (3) | 2.5 | 21.0 |

1) Previous solution acidified with sulphuric acid
2) 3-hydroxypropionitrile
3) Cu(I) complex generated *in situ* by recirculating Cu(II) solution over copper shot.

## Claims

1. A process for the removal of a olefin from a fluid, comprising

    (a) contacting a first solution containing copper (II) species with metallic copper to form an aqueous second solution containing copper (I) complexes wherein said second solution contains a complexing ligand selected from the group consisting of pyridine, methyl substituted pyridines, methoxy substituted pyridines, and compounds represented by the formulae

    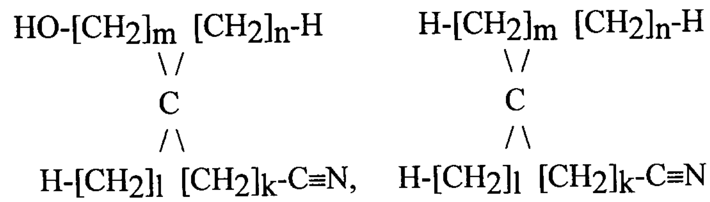

    where k = 0 to 4, l = 0 to 4, m = 0 to 4, n = 0 to 4, and combinations of the same; and
    (b) contacting the aqueous second solution containing copper (I) complexes with the olefin-containing fluid to remove a portion of the olefin from the fluid.

2. A process according to claim 1 wherein k + l + m + n = 0 to 4 .

3. A process according to claim 1 wherein k + l + m + n = 0 to 2 .

4. A process according to any one of the preceding claims wherein said second solution contains a neutral complexing ligand selected from the group consisting of pyridine, acetonitrile, 3-hydroxypropionitrile, and combinations of the same.

5. A process according to any one of the preceding claims wherein said copper(I) species is selected from the group consisting of cuprous acetate, cuprous nitrate, cuprous sulphate, and combinations of the same.

6. A process according to any one of the preceding claims wherein said second solution has a concentration of copper(I) complexes between about 1 M and about 6 M.

7. A process according to claim 6 wherein said second solution has a concentration of copper(I) complexes between about 2 M and about 3 M.

8. A process according to any one of the preceding claims wherein the ratio of molar concentration of the neutral complexing ligand to molar concentration of copper is between about 1 and about 6.

9. A process according to any one of the preceding claims wherein the ratio of molar concentration of the neutral complexing ligand to molar concentration of copper is between about 2 and about 3.

10. A process according to any one of the preceding claims wherein said neutral complexing ligand is pyridine.

11. A process according to claim 10 wherein the ratio of molar concentration of pyridine to the molar concentration of copper is between about 2 and about 3.

12. A process according to claim 10 or 11 wherein said second solution has a concentration of copper(I) complexes between about 1.9 M and about 3.0 M.

13. A process according to any one of the preceding claims wherein said olefin is selected from the group consisting of C2 hydrocarbons, C3 hydrocarbons, and combinations of the same.

14. A process according to claim 13 wherein said olefin is selected from the group consisting of ethylene, propylene, and combinations of the same.

**15.** A process according to claim 14 wherein said olefin is ethylene.

**Patentansprüche**

**1.** Verfahren zur Entfernung eines Olefins aus einem Fluid umfassend, daß man

a) eine erste Lösung, die Kupfer(II)molekülarten enthält, mit metallischem Kupfer in Kontakt bringt, um eine wäßrige zweite Lösung zu bilden, die Kupfer(I)komplexe enthält, wobei die zweite Lösung einen komplexbildenden Liganden enthält ausgewählt aus der Gruppe bestehend aus Pyridin, mit Methylgruppen substituierten Pyridinen, mit Methoxygruppen substituierten Pyridinen und Verbindungen der Formeln

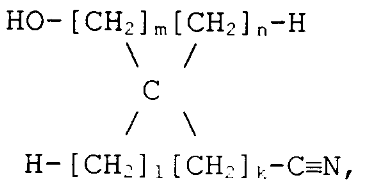

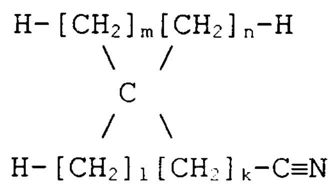

worin k 0 bis 4 ist, l 0 bis 4 ist, m 0 bis 4 ist, n 0 bis 4 ist und Kombinationen derselben und

b) die wäßrige zweite Lösung, die Kupfer(I)komplexe enthält, mit dem olefinhaltigen Fluid in Kontakt bringt, um einen Teil des Olefins aus dem Fluid zu entfernen.

**2.** Verfahren nach Anspruch 1, worin $k + l + m + n = 0$ bis 4 ist.

**3.** Verfahren nach Anspruch 1, worin $k + l + m + n = 0$ bis 2 ist.

**4.** Verfahren nach einem der vorhergehenden Ansprüche, worin die zweite Lösung einen neutralen komplexbildenden Liganden ausgewählt aus der Gruppe bestehend aus Pyridin, Acetonitril, 3-Hydroxypropionitril und Kombinationen davon enthält.

**5.** Verfahren nach einem der vorhergehenden Ansprüche, worin die Kupfer(I)molekülart ausgewählt ist aus der Gruppe bestehend aus Kupfer(I)acetat, Kupfer(I)nitrat, Kupfer(I)sulfat und Kombinationen davon.

**6.** Verfahren nach einem der vorhergehenden Ansprüche, worin die zweite Lösung eine Konzentration an Kupfer(I)komplexen von etwa 1 M bis etwa 6 M hat.

**7.** Verfahren nach Anspruch 6, worin die zweite Lösung eine Konzentration an Kupfer(I)komplexen von etwa 2 M bis etwa 3 M hat.

**8.** Verfahren nach einem der vorhergehenden Ansprüche, worin das Verhältnis der molaren Konzentration des neutralen komplexbildenden Liganden zu der molaren Konzentration von Kupfer zwischen etwa 1 und etwa 6 liegt.

**9.** Verfahren nach einem der vorhergehenden Ansprüche, worin das Verhältnis der molaren Konzentration des neutralen komplexbildenden Liganden zu der molaren Konzentration an Kupfer zwischen etwa 2 und etwa 3 liegt.

**10.** Verfahren nach einem der vorhergehenden Ansprüche, worin der neutrale komplexbildende Ligand Pyridin ist.

**11.** Verfahren nach Anspruch 10, worin das Verhältnis von molarer Konzentration an Pyridin zu molarer Konzentration an Kupfer zwischen etwa 2 und etwa 3 liegt.

**12.** Verfahren nach Anspruch 10 oder Anspruch 11, worin die zweite Lösung eine Konzentration an Kupfer(I)komplexen von etwa 1,9 M bis etwa 3,0 M hat.

**13.** Verfahren nach einem der vorhergehenden Ansprüche, worin das Olefin ausgewählt ist aus der Gruppe bestehend aus $C_2$-Kohlenwasserstoffen, $C_3$-Kohlenwasserstoffen und Kombinationen davon.

**14.** Verfahren nach Anspruch 13, worin das Olefin ausgewählt ist aus der Gruppe bestehend aus Ethylen, Propylen und Kombinationen davon.

**15.** Verfahren nach Anspruch 14, worin das Olefin Ethylen ist.

**Revendications**

**1.** Procédé pour éliminer une oléfine d'un fluide, comprenant

(a) la mise en contact d'une première solution contenant une entité de cuivre (II) avec du cuivre métallique pour former une seconde solution aqueuse contenant des complexes de cuivre (I), ladite seconde solution contenant un ligand complexant choisi dans le groupe consistant en pyridine, pyridines à substituant méthyle, pyridines à substituant méthoxy, et des composés représentés par les formules

```
HO-[CH2]m [CH2]n-H        H-[CH2]m [CH2]n-H
       \ /                       \ /
        C                         C
       / \                       / \
H-[CH2]l [CH2]k-C≡N,      H-[CH2]l [CH2]k-C≡N
```

dans lesquelles k a une valeur de 0 à 4, l à une valeur de 0 à 4, m a une valeur de 0 à 4, n a une valeur de 0 à 4, et leurs associations ; et

(b) la mise en contact de la seconde solution aqueuse contenant des complexes de cuivre (I) avec le fluide contenant une oléfine pour éliminer une portion de l'oléfine du fluide.

**2.** Procédé suivant la revendication 1, dans lequel la somme k+l+m+n a une valeur de 0 à 4 .

**3.** Procédé suivant la revendication 1, dans lequel la somme k+l+m+n a une valeur de 0 à 2 .

**4.** Procédé suivant l'une quelconque des revendications précédentes, dans lequel la seconde solution contient un ligand complexant neutre choisi dans le groupe consistant en pyridine, acétonitrile, 3-hydroxypropionitrile et leurs associations.

**5.** Procédé suivant l'une quelconque des revendications précédentes, dans lequel l'entité de cuivre (I) est choisie dans le groupe consistant en acétate cuivreux, nitrate cuivreux, sulfate cuivreux et leurs associations.

**6.** Procédé suivant l'une quelconque des revendications précédentes, dans lequel la seconde solution a une concentration de complexes de cuivre (I) comprise dans l'intervalle d'environ 1 M à environ 6 M.

**7.** Procédé suivant la revendication 6, dans lequel la seconde solution a une concentration en complexes de cuivre (I) comprise dans l'intervalle d'environ 2 M à environ 3 M.

**8.** Procédé suivant l'une quelconque des revendications précédentes, dans lequel le rapport de la concentration molaire du ligand complexant neutre à la concentration molaire du cuivre est compris dans l'intervalle d'environ 1 à environ 6.

**9.** Procédé suivant l'une quelconque des revendications précédentes, dans lequel le rapport de la concentration molaire du ligand complexant neutre à la concentration molaire du cuivre est compris dans l'intervalle d'environ 2 à environ 3.

**10.** Procédé suivant l'une quelconque des revendications précédentes, dans lequel le ligand complexant neutre est la pyridine.

**11.** Procédé suivant la revendication 10, dans lequel le rapport de la concentration molaire de la pyridine à la concentration molaire du cuivre est compris dans l'intervalle d'environ 2 à environ 3.

**12.** Procédé suivant la revendication 10 ou 11, dans lequel la seconde solution a une concentration en complexes de cuivre (I) comprise dans l'intervalle d'environ 1,9 M à environ 3,0 M.

**13.** Procédé suivant l'une quelconque des revendications précédentes, dans lequel l'oléfine est choisie dans le groupe consistant en hydrocarbures en $C_2$, hydrocarbures en $C_3$ et leurs associations.

**14.** Procédé suivant la revendication 13, dans lequel l'oléfine est choisie dans le groupe consistant en éthylène, propylène et leurs associations.

**15.** Procédé suivant la revendication 14, dans lequel l'oléfine consiste en éthylène.

FIG.1 : OLEFIN CAPACITY OF VARIOUS COMPLEXES